# EUROPÄISCHE PATENTSCHRIFT

(11) **EP 3 153 022 B1**
(45) Veröffentlichungstag und Bekanntmachung des Hinweises auf die Patenterteilung: **20.11.2019**
(21) Anmeldenummer: 15188445.9
(22) Anmeldetag: 06.10.2015
(51) Int. Cl.: C07D 231/14, A01N 43/56, A01P 3/00

(54) **N-(3',4'-DICHLOR-5-METHOXYBIPHENYL-2-YL)-3-(DIFLUORMETHYL)-1-METHYL-1H-PYRAZOL-4-CARBOXAMID**
N-(3',4'-DICHLOR-5-METHOXYBIPHENYL-2-YL)-3-(DIFLUORMETHYL)-1-METHYL-1H-PYRAZOL-4-CARBOXAMIDE
N-(3',4'-DICHLOR-5-METHOXYBIPHENYL-2-YL)-3-(DIFLUORMETHYL)-1-METHYL-1H-PYRAZOL-4-CARBOXAMIDE

(43) Veröffentlichungstag der Anmeldung: 12.04.2017
(73) Patentinhaber: Bayer CropScience Aktiengesellschaft, 40789 Monheim am Rhein (DE)
(72) Erfinder: Uroic, Kalle Miran, 50354 Hürth (DE); Himmler, Thomas, 51519 Odenthal (DE); Dahmen, Peter, 41470 Neuss (DE)
(74) Vertreter: BIP Patents

(56) Entgegenhaltungen:
- EP-A1- 2 071 953
- WO-A1-2007/128756

## Beschreibung

Die vorliegende Erfindung betrifft die Verbindung der Formel (I) 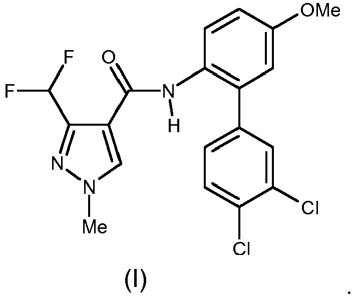 Weitere Gegenstände der Erfindung sind ein Mittel zur Bekämpfung von unerwünschten Mikroorganismen, ein Verfahren zur Bekämpfung von unerwünschten Mikroorganismen, die Verwendung der erfindungsgemäßen Verbindung oder eines erfindungsgemäßen Mittels zur Bekämpfung von unerwünschten Mikroorganismen, die Verwendung der erfindungsgemäßen Verbindung oder eines erfindungsgemäßen Mittels zum Schutz des Vermehrungsmaterials von Pflanzen, bei denen die chirurgische, therapeutische und diagnostische Behandlung des menschlichen oder tierischen Körpers ausgeschlossen ist und eine agrochemische Formulierung.

Da sich die ökologischen und ökonomischen Anforderungen an moderne Fungizide laufend erhöhen, beispielsweise was Wirkungsspektrum, Toxizität, Selektivität, Aufwandmenge, Rückstandsbildung und Herstellbarkeit angeht, und außerdem Probleme mit Resistenzen auftreten, besteht die Aufgabe, neue Fungizide zu entwickeln, die zumindest in Teilbereichen den genannten Anforderungen besser genügen. Diese Aufgabe wird durch die Verbindung der Formel (I) gelöst.

So wurde beispielsweise überraschenderweise gefunden, dass die erfindungsmäße Verbindung eine deutlich bessere präventive Wirksamkeit gegenüber *Blumeria* im Vergleich zu dem bekannten strukturell verwandten Fungizid Bixafen aufweist.

Die vorliegende Erfindung betrifft weiterhin ein Mittel zum Bekämpfen von unerwünschten Mikroorganismen, umfassend die erfindungsgemäße Verbindung.

Vorzugsweise handelt es sich um ein fungizides Mittel, das landwirtschaftlich verwendbare Hilfsmittel, Solventien, Trägerstoffe, oberflächenaktive Stoffe oder Streckmittel enthält.

Erfindungsgemäß ist ein Trägerstoff eine natürliche oder synthetische, organische oder anorganische Substanz, mit welchen die Verbindung zur besseren Anwendbarkeit, v.a. zum Aufbringen auf Pflanzen oder Pflanzenteile oder Saatgut, gemischt ist. Der Trägerstoff, der fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, die bei normaler Temperatur und unter Normaldruck gasförmig sind, z.B. Aerosol-Treibgase, wie Halogenkohlenwasserstoffe, sowie Butan, Propan, Stickstoff und Kohlendioxid.

In den erfindungsgemäßen Mitteln können Haftmittel wie Carboxymethylcellulose, natürliche und synthetische pulverige, körnige oder latexförmige Polymere, wie Gummiarabikum, Polyvinylalkohol, Polyvinylacetat, sowie natürliche Phospholipide, wie Kephaline und Lecithine, und synthetische Phospholipide verwendet werden. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile, wie z.B. oberflächenaktive Stoffe enthalten. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt bevorzugt zwischen 5 und 40 Gewichtsprozent des erfindungsgemäßen Mittels.

Es können auch Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten wie z.B. schützende Kolloide, Bindemittel, Klebstoffe, Verdicker, thixotrope Stoffe, Penetrationsförderer, Stabilisatoren, Sequestiermittel, Komplexbildner enthalten sein. Im Allgemeinen kann die Verbindung mit jedem festen oder flüssigen Additiv, welches für Formulierungszwecke gewöhnlich verwendet wird, kombiniert werden.

Im Allgemeinen enthalten die erfindungsgemäßen Mittel zwischen 0,05 und 99 Gewichtsprozent von der erfindungsgemäßen Verbindung, bevorzugt zwischen 0,1 und 95 Gewichtsprozent, besonders bevorzugt zwischen 0,5 und 90 Gewichtsprozent, ganz besonders bevorzugt zwischen 10 und 70 Gewichtsprozent.

Die erfindungsgemäße Verbindung bzw. die erfindungsgemäßen Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Mittel können in an sich bekannter Weise hergestellt werden, z.B. durch Vermischen der erfindungsgemäßen Verbindung mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäße Verbindung kann als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung: z.B. Benomyl, Carbendazim, Diethofencarb, Ethaboxam, Fuberidazole, Pencycuron, Thiabendazole, Thiophanate-methyl, Zoxamide;
3) Inhibitoren der Respiration (Atmungsketten-Inhibitoren):
   3.1) Inhibitoren am Komplex I der Atmungskette: z.B. Diflumetorim;
   3.2) Inhibitoren am Komplex II der Atmungskette: z.B. Bixafen, Boscalid, Carboxin, Fenfuram, Fluopyram, Flutolanil, Furametpyr, Furmecyclox, Mepronil, Oxycarboxin, Penf lufen, Penthiopyrad, Thifluzamide;
   3.3) Inhibitoren am Komplex III der Atmungskette: z.B. Amisulbrom, Azoxystrobin, Cyazofamid, Dimoxystrobin, Enestrobin, Famoxadone, Fenamidone, Fluoxastrobin, Kresoxim-methyl, Metominostrobin, Orysastrobin, Picoxystrobin, Pyraclostrobin, Trifloxystrobin;
4) Entkoppler: z.B. Dinocap, Fluazinam, Meptyldinocap;
5) Inhibotoren der ATP Produktion: z.B. Fentin acetate, Fentin chloride, Fentin hydroxide, Silthiofam;
6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Etridiazole, Iodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bitertanol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Myclobutanil, Naftifine, Nuarimol, Oxpoconazole, Paclobutrazol, Pefurazoate, Penconazole, Prochloraz, Propiconazole, Prothioconazole, Pyributicarb, Pyrifenox, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole;
10) Inhibitoren der Zellwandsynthese: z.B. Benthiavalicarb, Dimethomorph, Flumorph, Iprovalicarb, Mandipropamid, Polyoxins, Polyoxorim, Validamycin A;
11) Inhibitoren der Melanin-Biosynthese: z.B. Carpropamid, Diclocymet, Fenoxanil, Phthalide, Pyroquilon, Tricyclazole;
12) Resistenzinduktoren: z.B. Acibenzolar-S-methyl, Probenazole, Tiadinil;
13) Verbindungen mit Multisite-Aktivität: z.B. Bordeaux Mixture, Captafol, Captan, Chlorothalonil, Copper naphthenate, Copper oxide, Copper oxychloride, Kupferzubereitungen wie z.B. Kupferhydroxid, Kupfersulfat, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schwefelzubereitungen wie z.B. Calcium polysulphide, Thiram, Tolylfluanid, Zineb, Ziram;
14) eine Verbindung aus der folgenden Liste: (2E)-2-(2-{[6-(3-Chlor-2-methylphenoxy)-5-fluorpyrimidin-4-yl]oxy]phenyl)-2-(methoxyimino)-N-methylacetamid, (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, 1-(4-Chlorphenyl)-2-(1H-1,2,4-triazol-1-yl)cycloheptanol, 1-[(4-Methoxyphenoxy)methyl]-2,2-dimethylpropyl-1H-imidazol-1-carboxylat, 2-(4-Chlorphenyl)-N-{2-[3-methoxy-4-(prop-2-yn-1-yloxy)phenyl]ethyl}-2-(prop-2-yn-1-yloxy)acetamid, 2,3,5,6-Tetrachlor-4-(methylsulfonyl)pyridin, 2-Butoxy-6-iod-3-propyl-4H-chromen-4-on, 2-Chlor-N-(1,1,3-trimethyl-2,3-dihydro-1H-inden-4-yl)nicotinamid, 2-Phenylphenol und Salze davon, 3,4,5-Trichlorpyridin-2,6-dicarbonitril, 3-[5-(4-Chlorphenyl)-2,3-dimethylisoxazolidin-3-yl]pyridin, 5-Chlor-6-(2,4,6-trifluorphenyl)-N-[(1R)-1,2,2-trimethylpropyl]-[1,2,4]triazolo[l,5-a]pyrimidin-7-amin, 5-Chlor-7-(4-methylpiperidin-1-yl)-6-(2,4,6-trifluorphenyl)-[1,2,4]triazolo[1,5-a]pyrimidin, 5-Chlor-N-[(1R)-1,2-dimethylpropyl]-6-(2,4,6-trifluorphenyl)[1,2,4]triazolo[1,5-a]pyrimidin-7-amin, 8-Hydroxyquinolinsulfat, Benthiazol, Bethoxazin, Capsimycin, Carvone, Chinomethionat, Cufraneb, Cyflufenamid, Cymoxanil, Dazomet, Debacarb, Dichlorophen, Diclomezine, Dicloran, Difenzoquat, Difenzoquat Methylsulphate, Diphenylamine, Ferimzone, Flumetover, Fluopicolide, Fluoroimide, Flusulfamide, Fosetyl-Aluminium, Fosetyl-Calcium, Fosetyl-Natrium, Hexachlorbenzol, Irumamycin, Isotianil, Methasulfocarb, Methyl-(2E)-2-{2-[({cyclopropyl[(4-methoxyphenyl)imino]methyl}thio)methyl]phenyl}-3-methoxyacrylat, Methyl-1-(2,2-dimethyl-2,3-dihydro-1H-inden-1-yl)-1H-imidazol-5-carboxylat, Methyl-isothiocyanat, Metrafenone, Mildiomycin, N-[2-(1,3-Dimethylbutyl)phenyl]-5-fluor-1,3-dimethyl-1H-pyrazol-4-carboxamid, N-(3',4'-Dichlor-5-fluorbiphenyl-2-yl)-3-(difluormethyl)-1-methyl-1H-pyrazol-4-carboxamid, N-(3-Ethyl-3,5,5-trimethylcyclohexyl)-3-(formylamino)-2-hydroxybenzamid, N-(4-Chlor-2-nitrophenyl)-N-ethyl-4-methylbenzensulfonamide, N-(4-Chlorbenzyl)-3-[3-methoxy-4-(prop-2-yn-1-yloxy)phenyl]propanamid, N-[(4-Chlorphenyl)-(cyano)methyl]-3-[3-methoxy-4-(prop-2-yn-1-yloxy)phenyl]propanamid, N-[(5-Brom-3-chlorpyridin-2-yl)methyl]-2,4-dichlornicotinamid, N-[1-(5-Brom-3-chlorpyridin-2-yl)ethyl]-2,4-dichlomicotinamid, N-[1-(5-Brom-3-chlorpyridin-2-yl)ethyl]-2-fluor-4-iodnicotinamid, N-[2-(4-{[3-(4-Chlorphenyl)prop-2-yn-1-yl]oxy}-3-methoxyphenyl)ethyl]-N-(methylsulfonyl)valinamid, N-{(Z)-[(Cyclopropylmethoxy)imino][6-(difluormethoxy)-2,3-difluorphenyl]methyl}-2-phenylacetamid, N-{2-[1,1'-Bi(cyclopropyl)-2-yl]phenyl}-3-(difluormethyl)-1-methyl-1H-pyrazol-4-carboxamid, N-{2-[3-Chlor-5-(trifluormethyl)pyridin-2-yl]ethyl}-2-(trifluormethyl)benzamid, Natamycin, N-Ethyl-N-methyl-N'-{2-methyl-5-(trifluormethyl)-4-[3-(trimethylsilyl)propoxy]phenyl}imidoformamid, N-Ethyl-N-methyl-N'-{2-methyl-5-(difluormethyl)-4-[3-(trimethylsilyl)propoxy]phenyl}imidoformamid, Nickel dimethyldithiocarbamat, Nitrothal-isopropyl, O-{1-[(4-Methoxyphenoxy)methyl]-2,2-dimethylpropyl}1H-imidazol-1-carbothioat, Octhilinone, Oxamocarb, Oxyfenthiin, Pentachlorophenol und Salze davon, Phosphorsäure und Salze davon, Piperalin, Propamocarb Fosetylate, Propanosine-Natrium, Proquinazid, Pyribencarb, Pyrrolnitrine, Quintozene, S-Allyl-5-amino-2-isopropyl-4-(2-methylphenyl)-3-oxo-2,3-dihydro-1H-pyrazol-1-carbothioat, Tecloftalam, Tecnazene, Triazoxide, Trichlamide, Valiphenal, Zarilamid.

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/ -ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum))
   2.2 DDT
   2.3 Oxadiazine (z.B. Indoxacarb)
   2.4 Semicarbazon, zum Beispiel Metaflumizon (BAS320 I)
*3. Acetylcholin-Rezeptor-Agonisten*/*-Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad, Spinetoram)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor)
   5.2 Fiprole (z.B. Acetoprole, Ethiprole, Fipronil, Pyrafluprole, Pyriprole, Vaniliprole)
*6. Chlorid-Kanal-Aktivatoren*
   6.1 Mectine (z.B. Abamectin, Emamectin, Emamectin-benzoate, Ivermectin, Lepimectin, Milbemycin)
*7. Juvenilhormon-Mimetika*
   (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC, Meptyldinocap)
*12. Seite-I-Elektronentransportinhibitoren*
   12.1 METI's (z.B. Fenazaquin, Fenpyroximate, Flufenerim, Pyrimidifen, Pyridaben, Tebufenpyrad, Tolfenpyrad)
   12.2 Hydramethylnone
   12.3 Dicofol
*13. Seite-II-Elektronentransportinhibitoren*
   13.1 Cyenopyrafen, Cyflumetofen, Rotenone
*14. Seite-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofen, Spiromesifen)
   16.2 Tetramsäuren [z.B. Spirotetramat, cis-3-(2,5-dimethylphenyl)-4-hydroxy-8-methoxy-1-azaspiro-[4.5]dec-3-en-2-on]
*17. Oktopaminerge Agonisten*
   (z.B. Amitraz)
*18. Inhibitoren der Magnesium-stimulierten ATPase*
   18.1 Propargite
   18.2 Nereistoxin-Analoge (z.B. Thiocyclam hydrogen oxalate, Thiosultap-sodium)
*19. Agonisten des Ryanodin-Rezeptors,*
   19.1 Benzoesäuredicarboxamide [z.B. Flubendiamide]
   19.2 Anthranilamide [z.B. Chlorantraniliprole (Rynaxypyr, 3-bromo-N-{4-chloro-2-methyl-6-[(methyl-amino)carbonyl]phenyl} -1-(3-chloropyridin-2-yl)-1H-pyrazole-5-carboxamide)]
*20. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*21. Wirkstoffe mit unbekannten oder nicht spezifischen Wirkmechanismen*
   21.1 Begasungsmittel (z.B. Aluminium phosphide, Methyl bromide, Sulfuryl fluoride)
   21.2 Fraßhemmer (z.B. Flonicamid, Pymetrozine, Pyrifluquinazone)
   21.3 Milbenwachstumsinhibitoren (z.B. Clofentezine, Diflovidiazin, Etoxazole, Hexythiazox)
   21.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Dicyclanil, Fenoxacrim, Fentrifanil, Flubenzimine, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin.

Die erfindungsgemäße Behandlung der Pflanzen und Pflanzenteile mit der Verbindung bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Mikroorganismen. Bei diesen wird ein mit der Verbindung behandeltes Saatgut verwendet.

Die erfindungsgemäße Verbindung bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch eine nur kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit der erfindungsgemäßen Verbindung oder einem erfindungsgemäßen Mittel behandelt wird. Die Erfindung bezieht sich ebenfalls auf die Verwendung der erfindungsgemäßen Verbindung oder eines erfindungsgemäßen Mittels zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit der erfindungsgemäßen Verbindung oder einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Ebenso ist es als vorteilhaft anzusehen, dass die erfindungsgemäße Verbindung bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts können bereits durch die Expression des beispielsweise insektiziden Proteins bestimmte Schädlinge bekämpft werden.

Die erfindungsgemäße Verbindung und Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird die erfindungsgemäße Verbindung bzw. das Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge der auf das Saatgut aufgebrachten erfindungsgemäßen Verbindung bzw. des Mittels und/oder weiterer Zusatzstoffe so gewählt wird, dass die Keimung des Saatguts nicht beeinträchtigt bzw. die daraus hervorgehende Pflanze nicht geschädigt wird.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäße Verbindung kann in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindung mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatorenkommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung kommen alle üblicherweise für die Beizung einsetzbaren Mischgeräte in Betracht. Im Einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Verbindung bzw. Mittel weisen eine starke mikrobizide Wirkung auf und können zur Bekämpfung von unerwünschten Mikroorganismen, wie Pilzen und Bakterien, im Pflanzenschutz und im Materialschutz eingesetzt werden.

Gegenstand der Erfindung ist daher auch ein Verfahren zur Bekämpfung von unerwünschten Mikroorganismen, bei dem man die erfindungsgemäße Verbindung oder ein erfindungsgemäßes Mittel auf die unerwünschter Mikroorganismen und/oder ihren Lebensraum einwirken lässt.

Ebenfalls Gegenstand der Erfindung ist die Verwendung der erfindungsgemäßen Verbindung oder eines erfindungsgemäßen Mittels zur Bekämpfung von unerwünschten Mikroorganismen.

Bevorzugt sind bei dem erfindungsgemäßen Verfahren und / oder bei der erfindungsgemäßen Verwendung die chirurgische, therapeutische und diagnostische Behandlung des menschlichen oder tierischen Körpers ausgeschlossen.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Verbindung oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Verbindung. "Wirksame, aber nicht-phytotoxische Menge" bedeutet eine Menge der erfindungsgemäßen Verbindung, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise *Weißkohl, Rotkohl, Brokkoli, Blumenkohl, Rosenkohl, Pak Choi, Kohlrabi, Radieschen sowie Raps, Senf, Meerrettich und Kresse*)*, Fabacae sp. (beispielsweise Bohne, Erdnüsse), Papilionaceae sp. (beispielsweise Sojabohne), Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen. Bevorzugt werden Getreidepflanzen erfindungsgemäß behandelt.

Das erfindungsgemäße Verfahren zum Bekämpfen von phytopathogenen Pilzen kann auch zur Behandlung von genetisch veränderten Organismen, z.B. Pflanzen oder Samen, verwendet werden. Genetisch veränderte Pflanzen sind solche, in deren Genom ein bestimmtes heterologes Gen, welches für ein bestimmtes Protein codiert, stabil integriert wurde. "Heterologes Gen" meint dabei ein Gen, welches der transformierten Pflanze neue agronomische Eigenschaften verleiht, oder ein Gen, welches die agronomische Qualität der modifizierten Pflanze verbessert.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Verbindung und Mittel, besseres Pflanzenwachstum, weiter entwickeltes Wurzelsystem, höhere Beständigkeit der Pflanzenart bzw. Pflanzensorte, gesteigertes Wachstum der Schösslinge, höhere Pflanzenvitalität, erhöhte Toleranz gegenüber hohen oder niedrigen Temperaturen, erhöhte Toleranz ge-gen Trockenheit oder gegen Wasser- bzw. Bodensalzgehalt, erhöhte Blühleistung, erleichterte Ernte, Beschleunigung der Reife, höhere Ernteerträge, größere Früchte, höhere Pflanzengröße, grünere Blattfarbe, frühere Blüte, höhere Qualität und/oder höherer Ernährungswert der Ernteprodukte, höhere Zuckerkonzentration in den Früchten, höhere Lagerfähigkeit und/oder Bearbeitbarkeit der Ernteprodukte möglich, die über die eigentlich zu erwartenden Effekte hinausgehen.

Die erfindungsgemäße Verbindung weist auch eine stärkende Wirkung in Pflanzen auf. Sie eignet sich daher zur Mobilisierung pflanzeneigener Abwehrkräfte gegen Befall durch unerwünschte Mikroorganismen. Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die erfindungsgemäße Verbindung bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Verbindung zw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäße Verbindung bzw. die erfindungsgemäßen Mittel können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irreguläre, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Verbindung bzw. Mittel gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weist die erfindungsgemäße Verbindung der Formel (I) auch sehr gute antimykotische Wirkungen auf. Sie besitzt ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäße Verbindung kann daher sowohl in medizinischen als auch in nicht-medizinischen Anwendungen eingesetzt werden.

Beim Einsatz der erfindungsgemäßen Verbindung bzw. Mittel als Fungizide können die Aufwandmengen je nach Applikationsart innerhalb eines größeren Bereiches variiert werden. Die Aufwandmenge beträgt insbesondere
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen sind nur beispielhaft und nicht limitierend im Sinne der Erfindung genannt.

Die erfindungsgemäße Verbindung bzw. die erfindungsgemäßen Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die Verbindung der Formel (I) (N-(3',4'-Dichlor-5-methoxybiphenyl-2-yl)-3-(difluormethyl)-1-methyl-1H-pyrazol-4-carboxamid) 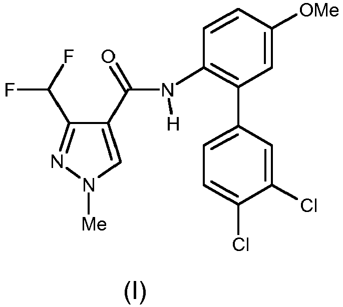 kann synthetisiert werden, indem man in einem ersten Schritt eine Verbindung der allgemeinen Formel (II) 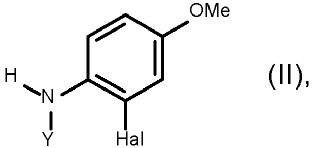 in der
- Y: für Wasserstoff oder einen Rest COR mit
- R: für Methyl, Ethyl, n-Propyl, i-Propyl, n-Butyl oder Phenyl
und
- Hal: für Brom oder Iod steht,
mit einer Verbindung der allgemeinen Formel (III) 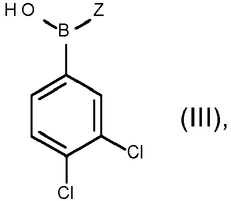 in welcher
- Z: für OH oder den Rest 3,4-Dichlorphenyl steht,
in Gegenwart eines Nickel- oder Palladiumkatalysators, eines Lösungsmittels und einer Base zu einer Verbindung der allgemeinen Formel (IV) umsetzt, 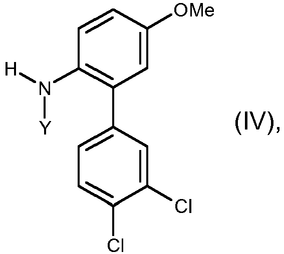 in der
- Y: die oben angegebenen Bedeutungen hat.
Bevorzugt setzt man eine Verbindung der allgemeinen Formel (II) 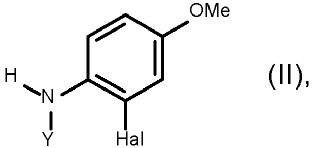 in der
- Y: für Wasserstoff oder einen Rest COR mit
- R: für Methyl
und
- Hal: für Brom steht
mit einer Verbindung der allgemeinen Formel (III) 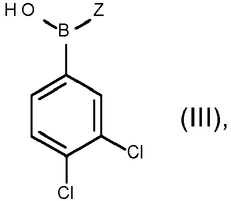 in welcher
- Z: für OH oder den Rest 3,4-Dichlorphenyl steht,
in Gegenwart eines Palladiumkatalysators, eines Lösungsmittels und einer Base zu einer Verbindung der allgemeinen Formel (IV) um 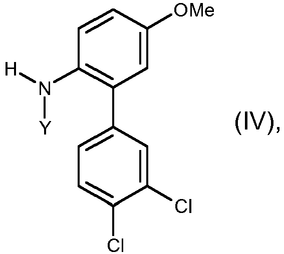 in der
- Y: die oben angegebenen Bedeutungen hat.

Als Lösungsmittel kommen alle unter den gewählten Reaktionsbedingungen inerten organischen Lösungsmittel sowie Wasser in Frage. Beispielhaft genannt seien Alkohole wie Methanol, Ethanol, n-Propanol, i-Propanol, n-Butanol; Kohlenwasserstoffe wie Toluol und Xylole; Nitrile wie Acetonitril; Formamide wie Dimethylformamid und Dimethylacetamid; Ketone wie Aceton, und 2-Butanon; Ether wie Tetrahydrofuran, 2-Methyltetrahydrofuran, Methyl-tertiärbutylether und 1,4-Dioxan; Wasser sowie ein- oder zweiphasige Gemische dieser Lösungsmittel.

Als Basen kommen organische und anorganische Basen in Frage. Beispielhaft genannt seien Triethylamin, Tributylamin, Pyridin, Picoline, Lutidine, Natriumcarbonat, Natriumhydrogencarbonat, Kaliumcarbonat, Kaliumhydrogencarbonat, Natriumphosphat, Natriumdihydrogenphosphat, Natriumhydrogenphosphat, Kaliumphosphat.

Die Basen werden für gewöhnlich in einem Überschuß, bezogen auf die Verbindung der Formel (III), eingesetzt, üblicherweise 1,1 bis 4 Äquivalente.

Als Palladiumkatalysator kann eine breite Palette von Palladium(0)-Komplexen und Palladium(II)-Salzen in Kombination mit Phosphinliganden eingesetzt werden. Beispielhaft seien genannt Pd(dba)₂ (dba = Dibenzylidenaceton), Pd₂(dba)₃, Pd(PPh₃)₄, Pd/C, PdCl₂, PdBr₂, Pd(acetylacetonat)₂, Pd(OAc)₂, Pd(NO₃)₂.

Der Palladiumkatalysator wird üblicherweise in Mengen zwischen 0,01 und 5 Molprozent, bezogen auf die Verbindung der Formel (II) eingesetzt.

Als Phosphinliganden seien beispielhaft genannt Triphenylphosphin, Tri(ortho-tolyl)phosphin, Tri(paramethoxyphenyl)phosphin und Tri(tertiärbutyl)phosphin.

Das molare Mengenverhältnis Ligand zu Palladium liegt üblicherweise im Bereich 1 bis 8.

Die Reaktionstemperatur für diesen Syntheseschritt liegt üblicherweise im Bereich von 20 bis 150°C.

Im Fall der Verbindung (IV) mit Y ungleich Wasserstoff wird in einem nächsten Schritt des Verfahrens diese Verbindung durch Hydrolyse nach allgemein bekannten und üblichen Methoden der organischen Chemie in die Verbindung der Formel (IV) mit Y gleich Wasserstoff überführt.

Im nächsten Schritt des Verfahrens wird die Verbindung der Formel (IV) mit Y gleich Wasserstoff mit einer Verbindung der allgemeinen Formel (V) 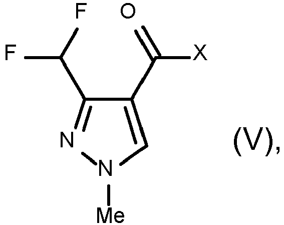 in der
- X: für Fluor oder Chlor steht,
in Gegenwart eines Lösungsmittels und gegebenenfalls einer Base zur Verbindung (I) umgesetzt.

Als Lösungsmittel kommen alle unter den gewählten Reaktionsbedingungen inerten organischen Lösungsmittel in Frage. Beispielhaft genannt seien Kohlenwasserstoffe wie Toluol, Xylole, Methylcyclohexan; Halogenkohlenwasserstoffe wie Methylenchlorid, Chlorbenzol, 1,2-Dichlorbenzol; Nitrile wie Acetonitril; Ketone wie Aceton, und 2-Butanon; Ether wie Tetrahydrofuran, 2-Methyltetrahydrofuran, Methyl-tertiärbutylether und 1,4-Dioxan; sowie Gemische dieser Lösungsmittel.

Als Basen kommen organische und anorganische Basen in Frage. Beispielhaft genannt seien Triethylamin, Tributylamin, Pyridin, Picoline, Lutidine, Natriumcarbonat, Natriumhydrogencarbonat, Kaliumcarbonat, Kaliumhydrogencarbonat, Natriumphosphat, Natriumdihydrogenphosphat, Natriumhydrogenphosphat.

Die Basen werden für gewöhnlich in einem Überschuß bezogen auf die Verbindung der Formel (IV) eingesetzt, üblicherweise 1,1 bis 4 Äquivalente.

Die Reaktion kann aber auch grundsätzlich ohne Verwendung einer Base durchgeführt werden.

Die Reaktion wird bei einer Temperatur zwischen 0 und 180°C durchgeführt.

Eine andere Möglichkeit zur Herstellung der Verbindung (I) besteht darin, zunächst eine Verbindung der allgemeinen Formel (II) 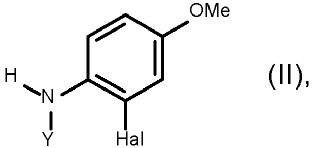 in der
- Y: für Wasserstoff steht,
- Hal: für Brom oder Iod steht,
mit einer Verbindung der allgemeinen Formel (V) 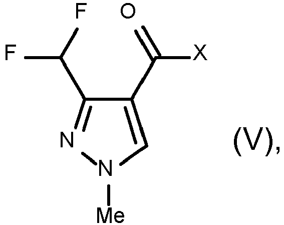 in der
- X: für Fluor oder Chlor steht,
in Gegenwart eines Lösungsmittels und gegebenenfalls einer Base zu einer Verbindung der allgemeinen Formel (VI) 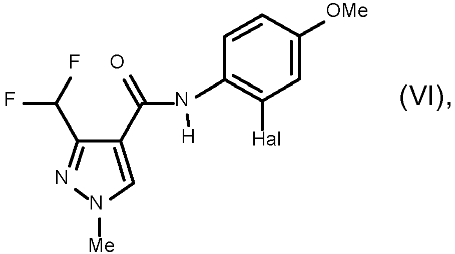 in der
- Hal: die oben angegebene Bedeutung hat,
umzusetzen.

In einem weiteren Schritt wird dann die Verbindung der allgemeinen Formel (VI) mit einer Verbindung der allgemeinen Formel (III) 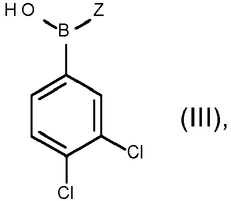 in welcher
- Z: für OH oder den Rest 3,4-Dichlorphenyl steht,
in Gegenwart eines Nickel- oder Palladiumkatalysators, eines Lösungsmittels und einer Base zur Verbindung (I) umgesetzt.

Die Herstellung der Verbindung der Formel (I) läßt sich demnach anhand der Schemata 1 und 2 veranschaulichen.

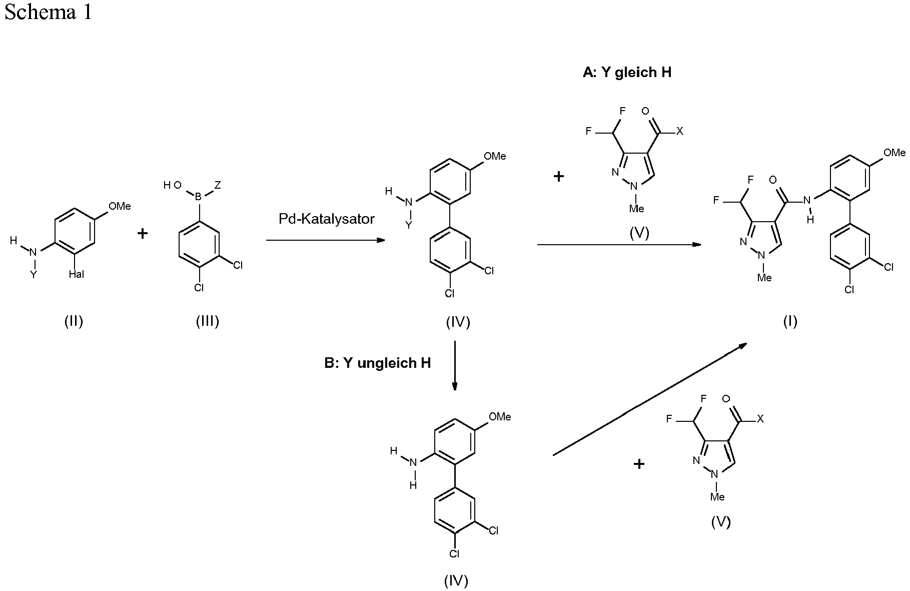

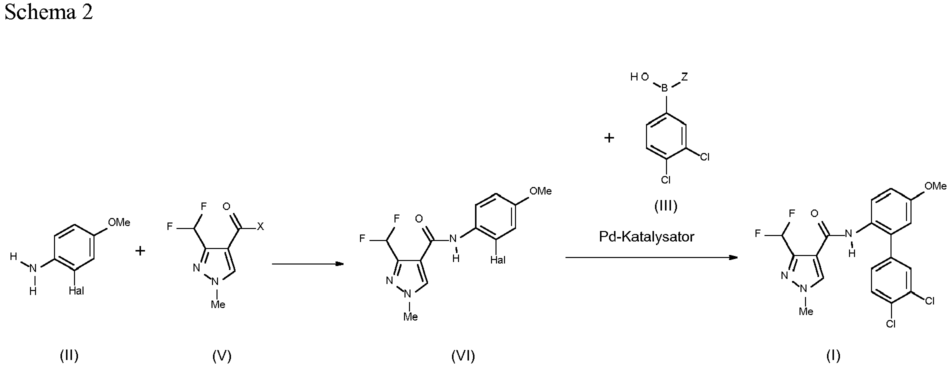

### Beispiele

### Beispiel 1: 3',4'-Dichlor-5-methoxybiphenyl-2-amin

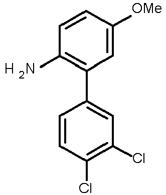

Die Reaktion wurde in luftfreien Lösungsmitteln unter einer Argonatmosphäre durchgeführt.

Zu einer Lösung von 3,017 g [14,8 mmol] 2-Brom-4-methoxy-anilin und 3,435 g [18 mmol] 3,4-Dichlorphenyl-boronsäure in einer Mischung aus 40 ml Toluol und 10 ml Ethanol wurde eine Lösung von 6,253 g [59 mmol] Na₂CO₃ in 29 ml Wasser gegeben. Nach Zugabe von 0,309 g [0,44 mmol] PdCl₂(PPh₃)₂ wurde das Reaktionsgemisch 16 Stunden bei 75°C gerührt. Danach wurde bei Raumtemperatur mit 50 ml Toluol und 50 ml Wasser verdünnt und über Celite filtriert. Die organische Phase wurde abgetrennt, mit 50 ml Wasser ausgeschüttelt, getrocknet und eingeengt.

Der erhaltene Rückstand wurde in 40 ml Petrolether aufgekocht. Nach Stehen über Nacht bei Raumtemperatur wurde der ausgefallene Feststoff abgesaugt, mit Petrolether gewaschen und getrocknet. Es wurden so 3,16 g der Zielverbindung erhalten (80% der Theorie).

LC/MS: m/e = 268 (MH⁺, 2 Cl³⁵)
GC/MS: m/e = 267 (M⁺, 2 Cl³⁵, 70%), 252 (M-15, 2 Cl³⁵, 100%).

### Beispiel 2: N-(3',4'-Dichlor-5-methoxybiphenyl-2-yl)-3-(difluormethyl)-1-methyl-1H-pyrazol-4-carboxamid

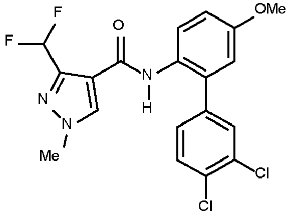

Zu einer Lösung von 2,789 g [10,4 mmol] 3',4'-Dichlor-5-methoxybiphenyl-2-amin und 1,578 g [15,6 mmol] Triethylamin in 50 ml Methylenchlorid wurde bei Raumtemperatur eine Lösung von 2,12 g [10,9 mmol] 3-(Difluormethyl)-1-methyl-1H-pyrazol-4-carbonylchlorid in 20 ml Methylenchlorid getropft. Nach 16 Stunden bei Raumtemperatur wurde das Reaktionsgemisch mit 100 ml Methylenchlorid verdünnt und mit 100 ml Wasser ausgeschüttelt. Die organische Phase wurde über Na₂SO₄ getrocknet und eingeengt. Der Rückstand wurde zweimal in Methyl-tertiärbutyl-ether (MTBE) aufgekocht und wieder abgesaugt, dann mit 1 N Salzsäure verrührt, abgesaugt und mit Wasser gewaschen. Anschließend wurde der so erhaltenen Rückstand mit einem Gemisch aus MTBE und Methylenchlorid (4:1) verrührt, abgesaugt, der Rückstand mit Ethanol verrührt, abgesaugt und getrocknet. Es resultierten 3,29 g Feststoff einer Reinheit von 96,9% (HPLC, a/a), womit sich eine Ausbeute von 74% der Theorie ergibt.

Schmelzpunkt: 186-188°C
LC/MS: m/e = 426 (MH⁺, 2 Cl³⁵)

¹H-NMR(600 MHz, CDCl₃): δ = 3,83 (s, 3H), 3,91 (s, 3H), 6,71 (t, J≈54 Hz, 1H), 6,78 (d, J≈3Hz, 1H), 6,96 (m, 1H), 7,21 (m, 1H), 7,48 (m, 2H), 7,65 (s(br), 1H), 7,87 (s, 1H), 7,92 ((d, J≈9Hz, 1H) ppm.

### Beispiel: in vivo präventive Blumeria Test (Gerste)

| | | |
|---|---|---|
| Lösungsmittel: | 49 | Gewichtsteile N,N-Dimethylacetamid |
| Emulgator: | 1 | Gewichtsteil Alkylarylpolyglycolether |

Um eine geeignete Zubereitung des Wirkstoffes herzustellen, wurde 1 Gewichtsteil des Wirkstoffs mit den angegebenen Mengen an Lösungsmittel und Emulgator gemischt und das Konzentrat mit Wasser auf die gewünschte Konzentration verdünnt.

Um die präventive Wirksamkeit zu prüfen, wurden junge Pflanzen mit der Zubereitung des Wirkstoffs mit der angegebenen Applikationsrate besprüht.

Nachdem die Sprühbeschichtung getrocknet worden war, wurden die Pflanzen mit Sporen von ***Blumeria graminis f.sp. hordei*** bestäubt.

Die Pflanzen wurden bei einer Temperatur von ca. 18 °C und einer relativen Luftfeuchtigkeit von ca. 80% in ein Gewächshaus gestellt, um die Entwicklung von Pilzpusteln zu fördern.

Der Test wurde nach 7 Tagen nach der Impfung ausgewertet. 0% bedeutete eine Wirksamkeit, die dem unbehandelten Vergleich entsprach, eine Wirksamkeit von 100% bedeutete, dass keine Erkrankung beobachtet wurde.

**Table: in vivo präventiv Blumeria Test (Gerste)**

| | Wirkstoff | Applikationsrate des Wirkstoffs in ppm | Wirksamkeit in % |
|---|---|---|---|
| Bixafen | 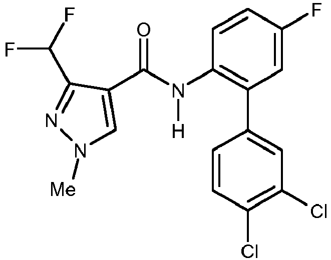 | 500 | 56 |
| | | 250 | 33 |
| | | 100 | 0 |
| Verbindung gemäß Formel (I) | 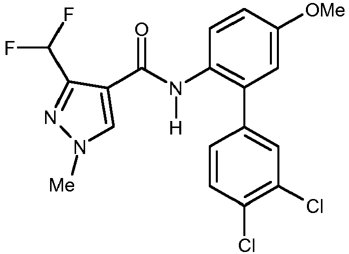 | 500 | 100 |
| | | 250 | 89 |
| | | 100 | 0 |

Der Test zeigt eine deutlich bessere präventive Wirksamkeit der erfindungsgemäßen Verbindung der Formel (I) gegenüber *Blumeria* im Vergleich zu dem bekannten, strukturell ähnlichen Fungizid Bixafen.

## Patentansprüche

1. Verbindung der Formel (I)

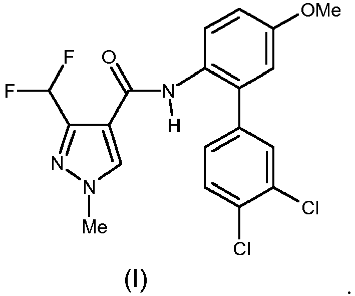

2. Mittel zum Bekämpfen von unerwünschter Mikroorganismen, umfassend die Verbindung gemäß Anspruch 1.

3. Verfahren zur Bekämpfung von unerwünschter Mikroorganismen, bei dem man die Verbindung gemäß Anspruch 1 oder ein Mittel gemäß Anspruch 2 auf die unerwünschter Mikroorganismen und/oder ihren Lebensraum einwirken läs st, und bei dem die chirurgische, therapeutische und diagnostische Behandlung des menschlichen oder tierischen Körpers ausgeschlossen ist.

4. Verwendung der Verbindung gemäß Anspruch 1 oder eines Mittels gemäß Anspruch 2 zur Bekämpfung von unerwünschter Mikro Organismen, bei der die chirurgische, therapeutische und diagnostische Behandlung des menschlichen oder tierischen Körpers ausgeschlossen ist.

5. Verwendung der Verbindung gemäß Anspruch 1 oder eines Mittels gemäß Anspruch 2 zum Schutz des Vermehrungsmaterials von Pflanzen.

6. Agrochemische Formulierung enthaltend die Verbindung gemäß Anspruch 1 in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

## Claims

1. Compound of the formula (I)

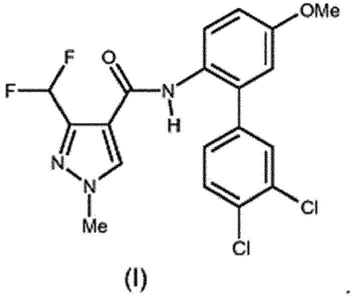

2. Composition for controlling undesirable microorganisms comprising the compound according to Claim 1.

3. Method for controlling undesirable microorganisms in which the compound according to Claim 1 or a composition according to Claim 2 is allowed to act on the undesirable microorganisms and/or their habitat, and in which the surgical, therapeutic and diagnostic treatment of the human or animal body is excluded.

4. Use of the compound according to Claim 1 or of a composition according to Claim 2 for controlling undesirable microorganisms in which the surgical, therapeutic and diagnostic treatment of the human or animal body is excluded.

5. Use of the compound according to Claim 1 or of a composition according to Claim 2 to protect the propagating material of plants.

6. Agrochemical formulation comprising the compound according to Claim 1 in biologically active contents of between 0.00000001 and 98% by weight, based on the weight of the agrochemical formulation, and also extenders and/or surface-active substances.

## Revendications

1. Composé de formule (I)

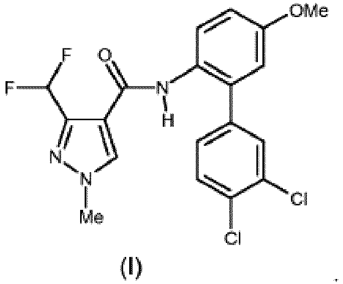

2. Agent pour la lutte contre des microorganismes indésirables, comprenant le composé selon la revendication 1.

3. Procédé pour la lutte contre des microorganismes indésirables, dans lequel on laisse agir le composé selon la revendication 1 ou un agent selon la revendication 2 sur les microorganismes indésirables et/ou leur lieu de vie, et dans lequel le traitement chirurgical, thérapeutique et de diagnostic du corps humain ou animal est exclus.

4. Utilisation du composé selon la revendication 1 ou d'un agent selon la revendication 2 pour la lutte contre des microorganismes indésirables, dans lequel le traitement chirurgical, thérapeutique et de diagnostic du corps humain ou animal est exclus.

5. Utilisation du composé selon la revendication 1 ou d'un agent selon la revendication 2 pour la protection du matériel de reproduction de plantes.

6. Formulation agrochimique contenant le composé selon la revendication 1 en des teneurs biologiquement actives comprises entre 0,00000001 et 98 % en poids, par rapport au poids de la formulation agrochimique, ainsi que des diluants et/ou des tensioactifs.
